# EUROPEAN PATENT APPLICATION

(11) **EP 4 702 912 A1**
(43) Date of publication of application: **04.03.2026**
(21) Application number: 24196799.1
(22) Date of filing: 27.08.2024
(51) Int. Cl.: A61B 1/00, A61B 1/12, A61M 1/00

(54) **A TUBING ASSEMBLY**

(71) Applicant: GA Health Medical Devices Limited, Mullingar Co. Westmeath N91 VEOH (IE)
(72) Inventor: McCabe, Ken, Hong Kong, NT (CN)
(74) Representative: Purdylucey Intellectual Property

(57) **Abstract**

An endoscopic tubing assembly (10) having irrigation tubing (20) configured to supply fluid from a fluid source (80) to an endoscope (30), the irrigation tubing (20) comprising an irrigation tube (40) having a distal fluid source end (55) and a proximal endoscope end (70); a fluid inlet (50) at the distal fluid source end (55), and a fluid outlet (60) and an irrigation tube connector (100) at the proximal endoscope end (70) for connecting the irrigation tube (40) to an endoscope (30) wherein a one-way valve (110) is provided on the irrigation tube upstream a distance D from the irrigation tube connector (100) between the irrigation tube connector (100) and the fluid source end (55).

## Description

### Introduction

This invention relates to a tubing assembly and more particularly to an endoscopic tubing assembly configured to supply fluids and/or suction to endoscopes during endoscopic procedures. The invention also relates to a method of preventing fluid from leaking from endoscope irrigation tubing and to a method of performing an endoscopic procedure.

### Background of the Invention

Endoscopes are used to allow surgeons to view internal organs and body passages of patients and are provided with a cable through which images are transmitted from a viewing means (e.g. a lens) to an imaging means visible to the surgeon. In addition, endoscopes are also provided with tubing assemblies, which can form part of or be in conjunction with the cable, through which fluids (liquids and/or gases) can pass for irrigation, insufflation, rinsing or other purposes. For example, the tubing assembly can include air/water tubing through which air/water can be directed towards the endoscope viewing means to wash debris from the viewing means. The tubing assembly can also include irrigation tubing from which water is forward directed to clear debris such as digestive waste, mucous and blood from the field of view e.g. from gastrointestinal mucosa and the like. The air/water tubing is generally connected to a pressurised water bottle and the irrigation tubing can also be connected to the pressurised water bottle or to a (peristaltic) pump which is also connected to a water bottle.

In many hybrid tubing assemblies a single water bottle can be employed to supply water to the air/water tubing and the irrigation tubing and the same water bottle can be employed for multiple patients generally in a twenty-four hour period. However, this practice presents a cross-contamination risk between patients as the tubing assembly and associated connectors can become contaminated with blood, stool and other body fluids that can travel back through the tubing assembly and into the water bottle. Accordingly, in order to mitigate this risk, known tubing assemblies are provided with backflow prevention mechanisms.

Typically, the backflow prevention mechanisms are made up of backflow-prevention or check valves, hereinafter referred to as one-way valves. For example, a one-way valve for the air/water tubing is generally located on the endoscope control handle while an irrigation tubing one-way valve is located away from the endoscope control handle and can be formed as part of a tube connector for connecting the irrigation tubing to the endoscope via an endoscope connector on the endoscope (which can also include a one-way valve).

However, it is known for water to leak from irrigation tubing at the tube connector/one-way valve following detachment of the irrigation tubing from the endoscope connector e.g. at the end of an endoscopic procedure. Such leakages can result in contamination, slippery floor surfaces, user falls and potential injuries.

An object of the invention is to overcome at least some of the problems of the prior art.

### Summary of the Invention

According to the invention there is provided an endoscopic tubing assembly having:
irrigation tubing configured to supply fluid from a fluid source to an endoscope, the irrigation tubing comprising an irrigation tube having a distal fluid source end and a proximal endoscope end;
a fluid inlet at the distal fluid source end, and
a fluid outlet and an irrigation tube connector at the proximal endoscope end for connecting the irrigation tube to an endoscope wherein
a one-way valve is provided on the irrigation tube upstream a distance D from the irrigation tube connector between the irrigation tube connector and the fluid source end.

In any embodiment, the one-way valve has a defined cracking pressure to only permit fluid having a pressure at or in excess of the cracking pressure to flow downstream through the one-way valve.

In any embodiment, a portion of the irrigation tube defined by the distance D is a coupling tube having the length D and a diameter d to fluidly couple the irrigation tube connector and the one-way valve.

In any embodiment, the coupling tube length D is set to control the fluid pressure of fluid in the coupling tube.

In any embodiment, the coupling tube diameter d is set to control the fluid pressure of fluid in the coupling tube.

In any embodiment, the coupling tube is attachable to and detachable from the irrigation tube.

In any embodiment, the tubing assembly is a hybrid tubing assembly further comprising air/water tubing.

In one embodiment, the fluid source is a pressurised water source.

In another embodiment, the fluid source is a pumped water source.

In another embodiment, the invention also extends to a method of preventing fluid from leaking from endoscope irrigation tubing comprising;
fluidly connecting an irrigation tube having a distal fluid source end and a proximal endoscope end to an upstream fluid source at the fluid source end and to a downstream endoscope at the proximal endoscope end, and
preventing fluid from flowing downstream from the distal fluid source end to the proximal endoscope end with a one-way valve, having a defined cracking pressure, located upstream a distance D from the proximal endoscope end between the endoscope end and the fluid source end unless the fluid has a pressure at or in excess of the cracking pressure, the portion of the irrigation tube defined by the distance D comprising a coupling tube having the length D and a diameter d.

In any embodiment, the method comprises fluidly connecting the proximal endoscope end of the irrigation tube to an endoscope with an irrigation tube connector.

In any embodiment, the method comprises setting the distance D to control the fluid pressure of fluid in the irrigation tube.

In any embodiment, the method comprises setting the diameter d of the coupling tube to control the fluid pressure of fluid in the irrigation tube.

In any embodiment, the upstream fluid source is a pressurised water source.

In any embodiment, the upstream fluid source is a pumped water source.

In a further aspect, the invention also extends to a method of performing an endoscopic procedure comprising connecting a fluid source to an endoscopic tubing assembly as hereinbefore defined, connecting the endoscopic tubing assembly to an endoscope and performing the endoscopic procedure.

In any embodiment, the method comprises fluidly connecting the proximal endoscope end of the irrigation tube to an endoscope with an irrigation tube connector.

In any embodiment, the method comprises setting the distance D to control the fluid pressure of fluid in the irrigation tube.

In any embodiment, the method comprises setting a diameter d of the coupling tube to control the fluid pressure of fluid in the irrigation tube.

In any embodiment, the fluid source is a pressurised water source.

In any embodiment, the fluid source is a pumped water source.

The tubing assembly of the invention only allows fluid to flow in one direction in an irrigation tube. The irrigation tube connector is located at the proximal endoscope end of the irrigation tube to connect to the endoscope, and the one-way valve having a defined or pre-determined cracking pressure is located a specific upstream distance from the irrigation tube connector so that only fluid having a pressure at or in excess of the cracking pressure is permitted to travel downstream through the one-way valve through the coupling tube. The one-way valve mechanism of the one-way valve ensures unidirectional fluid flow from the fluid source to the endoscope only e.g. when the gas inside a pressurized fluid container is pressurised to a certain level to force the fluid out of the fluid container to the irrigation tubing or where a peristaltic pump or similar exerts sufficient pressure on fluid from a container. Accordingly, since the fluid in the irrigation tubing can only flow through the one-way valve after achieving a specific pressure level, no fluid can flow through the one-way valve into the coupling tube and to the irrigation tube connector and the outlet until the desired pressure level has been achieved as determined by the cracking pressure of the one-way valve. This structure therefore stops any unintentional flow from the source of the fluid to the irrigation tube connector and hence prevents the problem of accidental dripping when the irrigation tubing is not connected to the endoscope. The method of the invention therefore prevents fluid from leaking from endoscope irrigation tubing at the conclusion of or following an endoscopic procedure.

### Brief Description of the Drawings

The invention will now be described, by way of example only, with reference to the accompanying drawings in which:

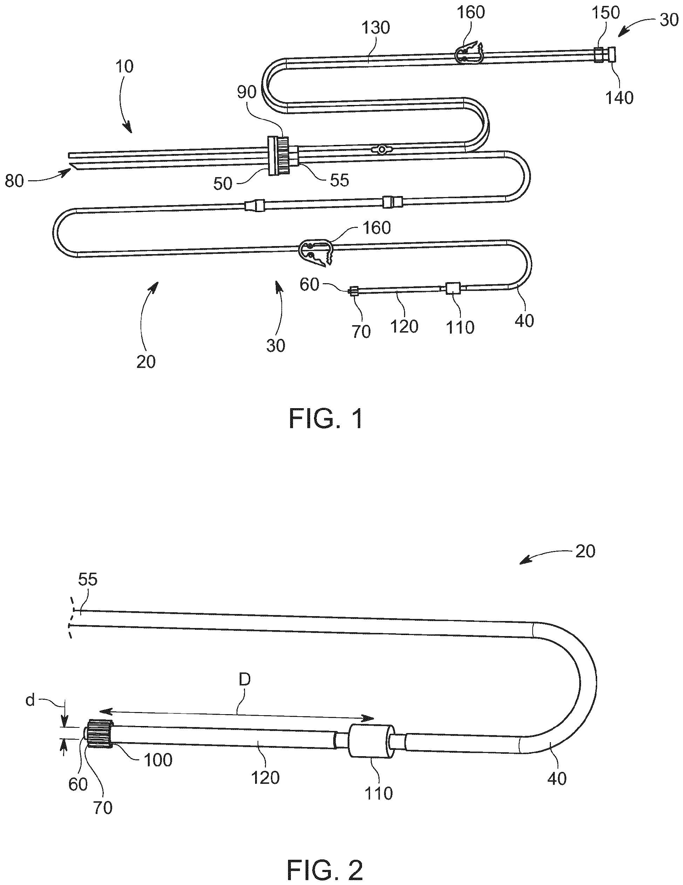

Figure 1 is a plan view from above of a hybrid tubing assembly of the invention in which the irrigation tubing is made up of an irrigation tube having a proximal endoscope end and a distal pressurised fluid source (e.g. a pressurised water bottle or pumped fluid) end in which the proximal endoscope end has a tube connector for connection to an endoscope (omitted for clarity) and a one-way valve is provided on the irrigation tube upstream from the tube connector towards the fluid source end of the irrigation tube, the one-way valve having a cracking pressure sufficient to prevent fluid flow through the one-way valve and hence the tube connector when the pressure applied to the pressurised fluid is below the cracking pressure e.g. when detaching the tube connector from an endoscope following depressurisation of the water bottle or deactivation of a fluid pump at the conclusion of an endoscopic procedure, and
Figure 2 is an enlarged plan view from above of the irrigation tubing of Figure 1.

### Detailed Description of the Invention

As shown in the drawings, an endoscopic tubing assembly in accordance with the invention is generally indicated by the reference numeral 10 and is made up of irrigation tubing 20 for clearing debris from the field of view of an endoscope (not shown but indicated by the reference numeral 30). The irrigation tubing 20 is formed from an irrigation tube 40 defining a fluid inlet 50 at a distal (pressurised) fluid source end 55 of the irrigation tube 40 and a fluid outlet 60 at a proximal endoscope end 70 of the irrigation tube 40. The fluid inlet 50 receives fluid into the irrigation tube 40 from a (pressurised) fluid chamber/source (not shown) indicated by the reference numeral 80 which can be for example a pressurised water bottle or pumped fluid from a water bottle. The irrigation tube 40 enters the pressurised fluid source 80 via a cap 90.

The proximal endoscope end 70 has a proximal irrigation tube connector 100 adjacent the fluid outlet 60 for connecting the irrigation tube 40 to the endoscope 30 and a distal one-way valve 110 on the irrigation tube 40 upstream from the tube connector 100 i.e. located towards the distal fluid source end 55 of the irrigation tube 40. The irrigation tube connector 100 and the one-way valve 110 are therefore spaced apart on the irrigation tube 40 by a distance D. The portion of the irrigation tube 40 defined by the distance D therefore fluidly couples the irrigation tube connector 100 with the distal one-way valve 110 and constitutes a coupling tube 120 having a length D. In the present embodiment, the coupling tube 120 is attachable to and detachable from the remainder of the irrigation tube 40. However, in other embodiments of the invention, the coupling tube 120 can be integral with the main body of the irrigation tube 40.

The one-way valve 110 has a cracking pressure sufficient to prevent downstream fluid flow through the one-way valve 110 and hence the irrigation tube connector 120 when the pressure applied to the pressurised fluid is below the cracking pressure e.g. when the irrigation tube connector 100 is detached from an endoscope 30 following an endoscopic procedure. Accordingly, the one-way valve 110 only lets fluid flow from the fluid chamber/source 80 to the fluid outlet 60 at the irrigation tube connector 100 when the fluid is pressurised to a certain level. Importantly, it also prevents any accidental flow from the source of the fluid 80 to the endoscope 30 e.g. following an endoscopic procedure. A suitable cracking pressure for the one-way valve 110 can range from about 1.1 bar to about 2.2 bar.

The length D of the coupling tube 120 together with the diameter d of the coupling tube 120 can be set/selected to vary, alternate and control the fluid pressure of fluid within the coupling tube 120 (which can vary according to the equipment employed) and therefore the force of the fluid exiting the fluid outlet 60 to the level required according to the endoscopic procedure being undertaken. This level of control of the pressurized fluid is not possible with the irrigation tube assemblies of the prior art where the one-way valve is located in the irrigation tube connector. By way of example, a suitable length D is from about 50mm to about 300mm and a suitable diameter d is from about 2mm to about 12mm.

In the present embodiment, the endoscopic tubing assembly 10 is a hybrid tubing assembly 10 in which air/water tubing 130 is also in fluid communication with the pressurized fluid source 80 via the cap 90. More particularly, the air/water tubing 130 includes an air/water tube 140 for conveying air/water from the fluid source 80 to the endoscope 30 via an air/water tube connector 150. However, as will be appreciated by those skilled in the art, in other embodiments, the tubing assembly 10 can comprise the irrigation tubing 20 only and/or other tubing as required.

As shown in the drawings, the tubing assembly 10 of the invention can also be provided with clips 160 on the irrigation tubing 20 and/or the air/water tubing 130 to secure the tubing where necessary.

In use, the tubing assembly 10 can be configured as shown in the drawings with the irrigation tubing 20 functioning as described below and the air/water tubing 130 functioning in a conventional manner familiar to those skilled in the art. The tubing assembly 10 is connected to the endoscope 30 by attaching the irrigation tubing 20 to the endoscope 30 via the irrigation tube connector 100 and a first endoscope connector (not shown) and the air/water tube to the endoscope 30 via a second endoscope connector (not shown). The fluid employed in the fluid source 80 and entering the endoscope 30 via the tubing assembly 10 should be appropriate to the procedure. The fluid in the fluid source 80 is then pressurized and drawn into the irrigation tube 40 of the irrigation tubing 20 via the fluid inlet 50. As indicated above, where a peristaltic pump is employed, the fluid is drawn into the irrigation tube 40 by the action of the peristaltic pump. The fluid then flows downstream through the irrigation tube 40 to the one-way valve 110 at the coupling tube 120. The one-way valve 110 prevents low pressure fluid having a pressure which is below that of the cracking pressure of the one-way valve 110 from flowing through the one-way valve 110. When the fluid has achieved the designated cracking pressure level of the one-way valve 110, the one-way valve opens to permit fluid flow through the coupling tube 120 to the irrigation tube connector 100 and through the fluid outlet 60 into the endoscope 30 as required during an endoscopic procedure. The fluid is then ejected from the endoscope 30 as required in conventional manner during endoscopic procedures.

Accordingly, fluid that flows out from the one-way valve 110 has a defined minimum or higher pressure according to the designated cracking pressure level. As indicated above, as the one-way valve 110 is situated at the pre-defined distance D from the irrigation tube connector 100 as determined by the length of the coupling tube 120, only fluid at a pressure that is higher than the cracking pressure will flow through the coupling tube 120 towards the irrigation tube connector 100 before entering the endoscope 30 via the outlet 60. Moreover, whilst flowing through the coupling tube 120, the fluid pressure is controlled and adjusted according to the length D and diameter d of the coupling tube 120 as required. Only fluid at the desired appropriate pressure then enters into the endoscope 30 via the irrigation tube connector 100.

## Claims

1. An endoscopic tubing assembly having:
irrigation tubing configured to supply fluid from a fluid source to an endoscope, the irrigation tubing comprising an irrigation tube having a distal fluid source end and a proximal endoscope end;
a fluid inlet at the distal fluid source end, and
a fluid outlet and an irrigation tube connector at the proximal endoscope end for connecting the irrigation tube to an endoscope wherein
a one-way valve is provided on the irrigation tube upstream a distance D from the irrigation tube connector between the irrigation tube connector and the fluid source end.

2. An endoscopic tubing assembly as claimed in Claim 1 wherein the one-way valve has a defined cracking pressure to only permit fluid having a pressure at or in excess of the cracking pressure to flow downstream through the one-way valve.

3. An endoscopic tubing assembly as claimed in Claim 1 or Claim 2 wherein a portion of the irrigation tube defined by the distance D is a coupling tube having the length D and a diameter d to fluidly couple the irrigation tube connector and the one-way valve.

4. An endoscopic tubing assembly as claimed in Claim 3 wherein the coupling tube length D is set to control the fluid pressure of fluid in the coupling tube.

5. An endoscopic tubing assembly as claimed in Claim 3 or Claim 4 wherein the coupling tube diameter d is set to control the fluid pressure of fluid in the coupling tube.

6. An endoscopic tubing assembly as claimed in any of Claims 3 to 5 wherein the coupling tube is attachable to and detachable from the irrigation tube.

7. An endoscopic tubing assembly as claimed in any of Claims 1 to 6 wherein the tubing assembly is a hybrid tubing assembly further comprising air/water tubing.

8. An endoscopic tubing assembly as claimed in any of Claims 1 to 7 wherein the fluid source is a pressurised water source.

9. An endoscopic tubing assembly as claimed in any of Claims 1 to 8 wherein the fluid source is a pumped water source.

10. A method of preventing fluid from leaking from endoscope irrigation tubing comprising;
fluidly connecting an irrigation tube having a distal fluid source end and a proximal endoscope end to an upstream fluid source at the fluid source end and to a downstream endoscope at the proximal endoscope end, and
preventing fluid from flowing downstream from the distal fluid source end to the proximal endoscope end with a one-way valve, having a defined cracking pressure, located upstream a distance D from the proximal endoscope end between the endoscope end and the fluid source end, unless the fluid has a pressure at or in excess of the cracking pressure, the portion of the irrigation tube defined by the distance D comprising a coupling tube having the length D and a diameter d.

11. A method as claimed in Claim 10 comprising fluidly connecting the proximal endoscope end of the irrigation tube to an endoscope with an irrigation tube connector.

12. A method as claimed in Claim 10 or Claim 11 comprising setting the distance D to control the fluid pressure of fluid in the irrigation tube.

13. A method as claimed in any of Claims 10 to 12 comprising setting the diameter d of the coupling tube to control the fluid pressure of fluid in the irrigation tube.

14. A method as claimed in any of Claims 10 to 13 wherein the upstream fluid source is a pressurised water source.

15. A method as claimed in any of Claims 10 to 14 wherein the upstream fluid source is a pumped water source.
